Europäisches Patentamt

**European Patent Office**

Office européen des brevets

(11) Numéro de publication: **0 161 144**
**B1**

(12) **FASCICULE DE BREVET EUROPEEN**

(45) Date de publication du fascicule du brevet:
22.03.89

(51) Int. Cl.⁴: **A 61 K 31/57**

(21) Numéro de dépôt: 85400612.9

(22) Date de dépôt: 28.03.85

(54) Composition pharmaceutique destinée à une administration orale constituée d'une part, à titre de principe actif, par la 9-alpha,11-bêta-dichloro 16-alpha-méthyl 21-oxycarbonyldicyclohexylméthyloxy pregna-1,4-diène 3,20-dione et d'autre part, par un excipient pharmaceutique inerte apte à permettre l'action du produit dans le rectum ou le colon et utilisation dudit principe actif dans la fabrication d'une telle composition.

(30) Priorité: 03.04.84 FR 8405222

(43) Date de publication de la demande:
13.11.85 Bulletin 85/46

(45) Mention de la délivrance du brevet:
22.03.89 Bulletin 89/12

(84) Etats contractants désignés:
AT BE CH DE FR GB IT LI LU NL SE

(56) Documents cités:
FR-A- 2 462 443
GB-A- 2 066 070

(73) Titulaire: ROUSSEL-UCLAF, 35, boulevard des Invalides,
F-75007 Paris (FR)

(72) Inventeur: Delevallée, Françoise, 48-50, avenue de la
Dame Blanche, F-94120 Fontenay-sous-Bois (FR)
Inventeur: Deraedt, Roger, 23, Allée Jean-Baptiste
Clément, F-93320 Les Pavillons-sous-Bois (FR)
Inventeur: Jouquey, Simone, 137, rue des Pyrénées,
F-75020 Paris (FR)

(74) Mandataire: Fritel, Hubert et al, Département des
Brevets ROUSSEL UCLAF B.P. no 9,
F-93230 Romainville (FR)

ACTORUM AG

## Description

La présente invention concerne une composition pharmaceutique destinée à une administration orale et constituée d'une part, à titre de principe actif, par la 9α,11β-dichloro 16α-méthyl 21-oxycarbonyldicyclohexyl méthyloxy pregna-1,4-diène 3,20-dione (produit A) et d'autre part, par un excipient pharmaceutique inerte, apte à permettre l'action du produit dans le rectum ou le colon.

Le produit A est décrit en tant que produit chimique dans le brevet français BF 2 462 443. Dans ce brevet, est également mentionnée pour ce produit, une activité anti-inflammatoire «in loco». Il est également indiqué dans ce brevet que le produit A est inactif par voie buccale et que la dissociation entre l'activité anti-inflammatoire «in loco» et l'activité par voie générale, notamment par voie buccale, est particulièrement intéressante pour l'utilisation en aérosol.

Les applications thérapeutiques envisagées dans le brevet français BF 2 462 443 sont ainsi l'asthme, les œdèmes, les dermatoses, les prurits, les diverses formes d'eczéma et les érythèmes solaires.

Dans la demande de brevet britannique GB-A-2 066 070 sont décrits de nouveaux comprimés à délitescence colique ainsi que leur procédé de préparation.

Or, la demanderesse vient de découvrir que le produit A présente une activité très intéressante dans les maladies inflammatoires du colon et du rectum et notamment dans la rectocolite hémorragique et la maladie de Crohn.

La présente invention a donc pour objet les compositions pharmaceutiques destinées à une administration orale et renfermant le produit A et aptes par leur excipient à permettre l'action de ce principe actif dans le colon ou le rectum. Ces compositions peuvent notamment se présenter sous les formes orales suivantes: comprimés simples ou drageifiés, gélules, granulés, capsules à délitescence colique.

Il semble bien que l'administration par voie orale permettrait au principe actif de franchir la plus grande partie du tube digestif sans transformation et d'atteindre ainsi le colon ou le rectum pour y apporter l'action recherchée.

Les compositions ci-dessus mentionnées peuvent être préparées selon les méthodes usuelles.

Le principe actif peut y être incorporé à des excipients habituellement employés dans les compositions pharmaceutiques, tels que le talc, le lactose, l'amidon et ses dérivés, la cellulose et ses dérivés, la polyvinylpyrrolidone, la silice colloïdale, les polyéthylèneglycols, le phosphate dicalcique, le phosphate tricalcique, les polymères de l'acide acrylique, les polymères de l'acide méthacrylique-méthacrylate, la gélatine, le stéarate de magnésium, les véhicules aqueux ou non, les corps gras d'origine animale, végétale ou synthétique, les dérivés paraffiniques, les glycols, les divers agents mouillants, dispersants ou émulsifiants, les conservateurs.

Les capsules à délitescence colique peuvent notamment être préparées selon la méthode indiquée dans le brevet français 2 471 186.

La posologie varie notamment en fonction du sujet à traiter et de l'affection en cause. Elle peut être comprise entre 1 et 100 mg/j plus spécialement entre 5 et 50 mg et plus particulièrement entre 20 à 50 mg/j.

A titre d'exemple, on a réalisé les gélules suivantes:
– Produit A                                   10 mg
– Excipient q.s. pour une gélule
  terminée à                                  120 mg
– Produit A                                   25 mg
– Excipient q.s. pour une gélule
  terminée à                                  150 mg

La présente invention a plus spécialement pour objet une composition pharmaceutique telle que décrite précédemment caractérisée en ce qu'elle est sous forme de comprimé ou de gélule.

Plus spécialement encore la composition pharmaceutique selon l'invention se présente sous forme de comprimés.

L'invention a enfin pour objet une utilisation de la 9α,11β-dichloro 16α-méthyl 21-oxycarbonyldicyclohexylméthyloxy pregna-1,4-diène 3,20-dione dans la fabrication d'une composition destinée par une administration orale à assurer l'action de ce principe actif dans le rectum ou le colon, ainsi qu'une utilisation de la 9α,11β-dichloro 16α-méthyl 21-oxycarbonyldicyclohexylméthyloxy pregna-1,4-diène 3,20-dione dans la fabrication d'une composition destinée par une administration orale à combattre les maladies inflammatoires du rectum et du colon et notamment la rectocolite hémorragique et la maladie de Crohn.

ETUDE PHARMACOLOGIQUE DE LA 9α,11β-DICHLORO 16α-METHYL 21-OXYCARBONYL DICYCLOHEXYL METHYLOXY PREGNA-1,4-DIENE 3,20-DIONE (produit A).

Des rats mâles Sprague-Dawley (200–250 g) sont sensibilisés par application cutanée d'une solution alcoolique de dinitrochlorobenzène (DNCB) à 2,5%. Après avoir sélectionné les animaux présentant au 7ème jour une réaction d'hypersensibilité positive, le test est pratiqué comme suit.

On administre quotidiennement pendant les 3 jours suivants par voie intrarectale, 2 ml d'une solution à 2,5% de DNCB dans un mélange acétone-huile de vaseline. En même temps, on administre par voie orale le produit à étudier.

Ces animaux sont sacrifiés 24 heures après le dernier traitement et la sévérité des lésions ulcéreuses est évaluée. Afin d'apprécier les éventuels effets systémiques des produits, on prélève également les thymus et les surrénales.

(Ce test est une modification de la méthode décrite par A.A.NORRIS Action of anti-colitic drugs on a guinea-pig model of immune colitis. Agents and Actions, 12 1/2, 239–242 (1982).

On a obtenu les résultats suivants:

| | Dose mg/kg Produit | % rats avec ulcères | Poids du thymus en mg | Poids des surré- nales en mg |
|---|---|---|---|---|
| Témoins | | 0 | 420 ± 24 | 40 ± 0,2 |
| DNCB | | 72 | 349 ± 44 | 45 ± 1,6 |
| DNCB + Produit A | 20 | 28 | 377 ± 24 | 42 ± 0,2 |
| DNCB ± Produit A | 50 | 14 | 345 ± 17 | 41 ± 1,5 |
| DNCB + Dexaméthasone | 0,1 | 72 | 117 ± 6** | 30 ± 1,7 |
| DNCB + Prednisolone | 50 | 100 | 109 ± 11** | 31 ± 2,7 |

Conclusion

Ces résultats montrent que le produit A présente une activité nette dans ce modèle de rectocolite immune sans effet systémique. Par contre, la dexaméthasone et la prednisolone se révèlent inactives contre les lésions ulcéreuses provoquées par le DNCB mais présentent une activité systémique comme en témoigne la diminution du poids du thymus et des surrénales.

**Revendications**

1. Composition pharmaceutique destinée à une administration orale et comportant d'une part, à titre de principe actif, la
9α,11β-dichloro 16α-méthyl 21-oxycarbonyl-dicyclohexylméthyloxy pregna-1,4-diène 3,20-dione
et d'autre part, un excipient pharmaceutique inerte, apte à permettre l'action du principe actif dans le rectum ou le colon.

2. Composition pharmaceutique selon la revendication 1, caractérisée en ce qu'elle se présente sous forme de comprimé ou de gélule.

3. Composition pharmaceutique selon la revendication 1 ou 2, pour son utilisation dans le traitement des maladies inflammatoires du colon et du rectum.

4. Composition pharmaceutique selon la revendication 1 ou 3, pour son utilisation dans le traitement de la rectocolite hémorragique et de la maladie de Crohn.

5. Utilisation de la
9α,11β-dichloro 16α-méthyl 21-oxycarbonyl-dicyclohexylméthyloxy pregna-1,4-diène 3,20-dione
dans la fabrication d'une composition destinée par une administration orale à assurer l'action de ce principe actif dans le rectum ou le colon.

6. Utilisation de la
9α,11β-dichloro 16α-méthyl 21-oxycarbonyl-dicyclohexyl-méthyloxy pregna-1,4-diène 3,20-dione
dans la fabrication d'une composition destinée par une administration orale à combattre les maladies inflammatoires du rectum et du colon.

7. Utilisation de la
9α,11β-dichloro 16α-méthyl 21-oxycarbonyl-dicyclohexyl-méthyloxy pregna-1,4-diène 3,20-dione
dans la fabrication d'une composition destinée par une administration orale à combattre la rectocolite hémorragique et la maladie de Crohn.

**Claims**

1. Pharmaceutical composition intended for oral administration and containing on one hand, as active principle,
9 alpha, 11 beta-dichloro 16 alpha-methyl 21-oxycarbonyldicyclohexylmethyloxy pregna-1,4-diene 3,20-dione
and on the other hand, an inert pharmaceutical excipient, capable of allowing the action of the active principle in the rectum or colon.

2. Pharmaceutical composition according to claim 1, characterised in that it is presented in the form of a tablet or gelule.

3. Pharmaceutical composition according to claims 1 or 2 for its use in the treatment of inflammatory diseases of the colon and of the rectum.

4. Pharmaceutical composition according to claims 1 or 3, for its use in the treatment of haemorrhaging rectocolitis and Crohn's disease.

5. Use of
9 alpha, 11 beta-dichloro 16 alpha-methyl 21-oxycarbonyldicyclohexylmethyloxy pregna-1,4-diene 3,20-dione
in the manufacture of a composition intended, by oral administration, to ensure the action of this active principle in the rectum or colon.

6. Use of
9 alpha, 11 beta-dichloro 16 alpha-methyl 21-oxycarbonyldicyclohexyl-methyloxy pregna-1,4-diene 3,20 dione
in the manufacture of a composition intended, by oral administration, to combat inflammatory diseases of the rectum and colon.

7. Use of
9 alpha, 11 beta-dichloro 16 alpha-methyl 21-oxycarbonyldicyclohexyl-methoxy pregna-1,4-diene 3,20-dione
in the manufacture of a composition intended, by oral administration to combat haemorrhaging rectocolitis and Crohn's disease.

**Patentansprüche**

1. Pharmazeutische Zusammensetzung zur oralen Verabreichung, enthaltend einesteils

9α,11β-Dichlor-16α-methyl-21-oxycarbonyl-dicyclohexylmethyloxy-pregna-1,4-dien-3,20-dion

als Wirkstoff und anderenteils einen inerten, pharmazeutischen Exzipienten, der befähigt ist, die Entfaltung der Wirksamkeit des Wirkstoffs in dem Rektum oder dem Kolon zu ermöglichen.

2. Pharmazeutische Zusammensetzung gemäß Anspruch 1, dadurch gekennzeichnet, daß sie in Form von Tabletten oder Gelkapseln vorliegt.

3. Pharmazeutische Zusammensetzung gemäss Anspruch 1 oder 2 für deren Verwendung bei der Behandlung von entzündlichen Erkrankungen des Kolons und des Rektums.

4. Pharmazeutische Zusammensetzung gemäß Anspruch 1 oder 3 für deren Verwendung bei der Behandlung der hämorrhagischen Rektokolitis und der Crohn'schen Erkrankung.

5. Verwendung von 9α,11β-Dichlor-16α-methyl-21-oxycarbonyl-dicyclohexylmethyloxy-pregna-1,4-dien-3,20-dion bei der Herstellung einer Zusammensetzung zur oralen Verabreichung, um die Wirkung des Wirkstoffs in dem Rektum oder dem Kolon zu gewährleisten.

6. Verwendung von 9α,11β-Dichlor-16α-methyl-21-oxycarbonyl-dicyclohexylmethyloxy-pregna-1,4-dien-3,20-dion bei der Herstellung einer Zusammensetzung zur oralen Verabreichung, um entzündliche Erkrankungen des Rektums und des Kolons zu bekämpfen.

7. Verwendung von 9α,11β-Dichlor-16α-methyl-21-oxycarbonyl-dicyclohexylmethyloxy-pregna-1,4-dien-3,20-dion bei der Herstellung einer Zusammensetzung zur oralen Verabreichung, um die hämorrhagische Rektokolitis und die Crohn'sche Erkrankung zu bekämpfen.